# EUROPEAN PATENT APPLICATION

(11) **EP 4 789 646 A1**
(43) Date of publication of application: **12.08.2026**
(21) Application number: 25156964.6
(22) Date of filing: 10.02.2025
(51) Int. Cl.: A61F 13/20, A61F 5/455, A61N 1/05

(54) **INTRAVAGINAL DEVICE FOR TREATING PELVIC PAIN AND FOR BLOCKING VAGINAL FLUIDS**

(71) Applicant: Mughal, Idris, 1030 Schaerbeek (BE)
(72) Inventor: MUGHAL, Idris, 1030 Schaerbeek (BE); PODVIN, Antoine, 1180 Uccle (BE); MADDEN, Philip, 2930 Brasschaat (BE); COUCKE, Victor, 3001 Heverlee (BE); RUYTJENS, Tim, 2640 Mortsel (BE); VANSCHOENWINKEL, Martijn, 3140 Keerbergen (BE)
(74) Representative: Calysta NV

(57) **Abstract**

Intravaginal device for treating pelvic pain and for blocking fluids through a vagina, the intravaginal device comprises: a body configured to be placed in a vagina, the body comprising a ring-shaped rim configured to seal the body against the vaginal wall, when placed in the vagina, the body comprising a closure mean closing the surface area within the ring-shaped rim to block fluids through the vagina; and electrostimulation means configured to stimulate the vaginal wall to treat pelvic pain.

## Description

### Technical Field

The present invention relates to a Intravaginal device for treating pelvic pain and for blocking vaginal fluids

### Prior art

Menstrual pain and menstrual bleeding are common challenges faced by many women during their reproductive years, impacting their quality of life. Pain, often caused by uterine contractions (dysmenorrhea), can range from mild discomfort to debilitating cramps, interfering with daily activities, work, and mental well-being. Menstrual bleeding, more generally menstrual flow or liquids, can cause physical discomfort, inconvenience, and emotional strain, often disrupting daily activities and overall well-being.

The problem of menstrual fluids is normally managed by the means of menstrual higenic products. These menstrual higenic products can be broadly categorized in absorbing higenic products like tampons, sanitary/menstrual pads, pantyliners or period underwear and collecting higenic products like menstrual cups, menstrual discs. Menstrual higenic products can be further categorized in extravaginal products like sanitary/menstrual pads, pantyliners or period underwear and intravaginal products like tampons, menstrual cups and menstrual discs.

The menstrual pain can be treated in many ways like by general pain medication, hormonal control, heat therapy or other ways. Another way to treat pelvic pain in general is via electrostimulation as disclosed for example in WO2022/086893A1. However, such pelvic pain therapy devices are difficult to use during menstruation.

Therefore, it was proposed to incorporate such electrostimulation therapy in tampons like in WO2024/011123A1 or WO00/07658A1. However, even if this idea is known since many years, no working product has been reached for combining a pelvis treatment device with a absorbin intravaginal menstrual hygenic device.

### Brief summary of the invention

It is the object of the invention to provide a device which improves the pelvic pain reduction and the vaginal hygene of women.

It is further the object of the invention to provide a device which eases the challenges faced by many women during their menstrual cycle.

According to the invention, this object is solved by an intravaginal device for treating pelvic pain and for blocking fluids through a vagina, the intravaginal device comprises: a body configured to be placed in a vagina, the body comprising a ring-shaped rim configured to seal the body against the vaginal wall, when placed in the vagina, the body comprising a closure mean closing the surface area within the ring-shaped rim to block fluids through the vagina; and an means configured to stimulate the vaginal wall to treat pelvic pain.

By integrating the electrostimulation against pelvic pain in an intravaginal device which stops vaginal fluids has the advantage, that the same device which is used for blocking the fluids in the vagina and is placed there normally for long times can thus be used continuously to treat the pelvis pain. The body with the rim allows to seal the vaginal channel without the need of an absorbing material which allows to reuse the intravaginal device many times. Therefore, the electronics for the electrostimulation for the pelvic pain treatment can also be used many times.

According to the invention, this object is solved by a collecting menstrual hygiene device configured for treating pelvic pain and for collecting vaginal fluids, the device comprises: a body having a shape configured to be placed in a vagina at the vaginal wall and to collect vaginal fluid and prevent the collected vaginal fluid from leaving the vagina; and electrostimulation means configured to stimulate the vaginal wall to treat pelvic pain.

By integrating the electrostimulation against pelvic pain in a menstrual hygiene device has the advantage that the device for collecting the menstrual fluids can treat the menstrual pelvic pain effectively by electrostimulation. Since the menstrual hygiene device is of the collecting type (not of the absorbing type), the device is reusable. By integrating the pelvic pain treatment in such a collecting menstrual hygiene device allows to reuse the electronics for the electrostimulation for the pelvic pain treatment many times. The dependant claims refer to further advantageous embodiments.

In one embodiment, the body comprises a ring-shaped rim configured to be placed/pushed against a vaginal wall of the vagina and a blocking means in the space enclosed by the rim (to block the passage through the vagina at the position of the rim in the vaginal channel).

In one embodiment, the intravaginal device being a collecting menstrual hygiene device (in contrast to an absorbing menstrual hygiene device). Collecting menstrual hygiene devices can be reused (contrary to absorbing menstrual hygiene devices like tampons). This allows to reuse also the electronics for treating pelvic pain in the (multi-usage) collecting menstrual hygiene device several also multiple times. In a absorbing menstrual device, the electronics, if inserted in the tampon can normally be used only once and is thus disposable.

In one embodiment, the closure means forms a receptable to collect vaginal fluids. In one embodiment, the closure mean closing the surface area within the ring-shaped rim to block fluids through the vagina is a receptable or container closing the surface area within the ring-shaped rim to block vaginal fluids leaving the vagina and collecting the vaginal fluids.

In one embodiment, the intravaginal device or menstrual hygiene device being a menstrual disc. The menstrual disc has compared to a tampon or a menstrual cup the advantage that its position at one end is in the posterior fornix which is the optimal position to treat the pelvic pain with the electrostimulation. This is why preferably, at least one, preferably two of the electrodes are arranged at a first side opposed of the second side with a removal protrusion.

In one embodiment, the intravaginal device or menstrual hygiene device is a menstrual cup.

In one embodiment, the closure means forms a receptable to collect vaginal fluids.

In one embodiment, the closure means or body has a diameter which is larger than its depth.

In one embodiment, the closure means or body has a diameter smaller than 80 mm, preferably than 70mm and/or larger than 50mm. These are suitable dimensions to position the intravaginal device in the fornix, preferably with the electrodes in the posterior fornix.

In one embodiment, the body is designed such that a first side of the rim can be positioned at a fornix of the vagina, preferably a posterior fornix of the vagina. In this position, the body seals the vagina for the passage of fluids into the vagina or out of the vagina.

In one embodiment, the body comprises a removal protrusion for facilitating the removal of the intravaginal device.

In one embodiment, the removal protrusion is arranged at a side opposed to the electrodes.

In one embodiment, the removal protrusion protrudes from the rim. In one embodiment, removal protrusion protrudes radially to a receptable axis from the rim. In one embodiment, removal protrusion protrudes substantially parallel to a receptable axis from the rim. In one embodiment, the removal protrusion is attached bendable at the rim so that the angle between removal protrusion and an axis of the receptable is changeable

In one embodiment, the removal protrusion protrudes radially to a receptable axis from a bottom of the receptable.

In one embodiment, the removal protrusion protrudes centrally from a bottom of the receptable.

In one embodiment, the electrostimulation means comprises at least one, preferably two electrodes arranged in the rim. Since the rim presses against the vaginal wall to obtain a sealing effect and to block the fluids in the vagina, the contact with the vaginal wall is particular well. By arranging the electrodes in the rim which seals the vagina, not only are two functions combined in the same device, but also the electrostimulation is improved as the force of the rim again the vaginal wall to exert the sealing effect also improves the contact to the vaginal wall for the electrostimulation and thus improves the treatment of the pelvic pain.

In one embodiment, the body is made off silicon, wherein the at least one electrode is realized in conductive silicon embedded in the silicon of the body.

In one embodiment, the electrostimulation means comprises a power source, electronics and a conductor means, wherein the conductor means are configured to connect the electronics with the at least one electrode, wherein the electronics are configured to receive the power from the power source and to generate an electrical stimulation signal at the at least one electrode to stimulate the vaginal wall.

In one embodiment, the conductor means extends along the rim.

In one embodiment, the conductor means extends from the bottom of the receptable to the rim at the position of the at least one electrode.

In one embodiment, the conductor means is realized for each electrode as a conductor track on a flexible printed circuit board.

In one embodiment, the conductor means and the electronics are realized on the same flexible printed circuit board.

In one embodiment, the conductor means and the electronics are realized on a rigid-flex printed circuit board with at least some parts of the electronics being on the rigid part of the printed circuit board and at least the conductor means being realized in the flexible parts of the printed circuit board.

In one embodiment, the power source is arranged in the bottom of the receptable.

In one embodiment, the power source is arranged in the rim or the lateral wall of the receptable.

In one embodiment, the power source is a battery and the electrostimulation means comprises a charging circuit for wireless charging of the battery.

In one embodiment, the electronics are arranged in the removal protrusion.

In one embodiment, the electronics is arranged in rim opposite to the at least one electrode.

In one embodiment, removal protrusion is arranged opposed to the at least one electrode.

Other embodiments according to the present invention are mentioned in the appended claims and the subsequent description of an embodiment of the invention.

### Brief description of the Drawings

Fig. 1 shows a first embodiment of the intravaginal device according to the invetion.
Fig. 2 shows a second embodiment of the intravaginal device according to the invetion.
Fig. 3 shows a variation of the first embodiment of the intravaginal device according to the invetion.
Fig. 4 shows a variation of the second embodiment of the intravaginal device according to the invetion.
Fig. 5 shows a third embodiment of the intravaginal device according to the invetion.
Fig. 6 shows a fourth embodiment of the intravaginal device according to the invetion.
Fig. 7 shows schematically the position of the intravaginal device of Fig. 10 in a vagina.
Fig. 8 shows schematically the position of the intravaginal device of Fig. 3 in a vagina.
Fig. 9 shows a fifth embodiment of the intravaginal device according to the invetion.
Fig. 10 shows a sixth embodiment of the intravaginal device according to the invetion.
Fig. 11 shows a seventh embodiment of the intravaginal device according to the invetion.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

Fluids in the vagina could be vaginal fluids or extra-vaginal fluids. Vaginal fluids are all fluids produced by the vagina. During the menstrual cycle, the vaginal fluids contain menstrual fluids. However, vaginal fluids can also contain other vaginal fluids like cervical mucus, normal vaginal discharge or other. Extra-vaginal fluids could be any fluid entered from outside the vagina, for example sperma during sexual intercourse.

The intravaginal device according to the invention is configured to fulfill two purposes, first to treat pelvic pain by electrostimulation and second, to be positioned/ to seal such in the vagina that fluids cannot any more enter or leave the vagina. Preferably, the second functoin is to be positioned such in the vagina that vaginal fluids cannot leave the vagina, especially for preventing menstual fluids to leave the vagina. Preferably, the intravaginal device is a intravaginal hygiene device, preferably a intravaginal hygien device of the collecting type, also called a intravaginal collecting hygiene device. The intravaginal hygien device is preferably a menstrual hygien device but could used equally for collecting other vaginal fluids.

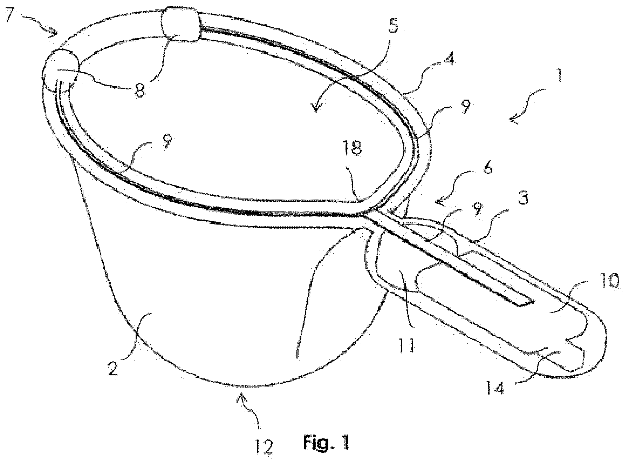

Embodiments of the the intravaginal collecting hygiene device 1, 1' are shown in Fig. 1 to 11. Before describing each embodiment in more detail, the intravaginal collecting hygiene device

The intravaginal device 1, 1' comprises a body and an electrostimulation means.

The body is configured to be placed in a vagina. The body is configured to seal the body against the vaginal wall 28, when placed in the vagina in a sealing position, and to block the passage of fluids through the vagina at the sealing position. The body is preferably elastic so that it can be inserted in the vagina and can adapt to the form of the vagina in the sealing position. The body is preferably made of an elastic material, preferably silicon. Obviously other elastic and non elastic materials are possible as well. If the body is elastic, all description refers to the non-compressed form of the elastic body, i.e. to its form/shape without any external influence.

The body extends preferably in a horizontal plane and a vertical direction. The vertical direction can be distinguihsed in a top direction and a bottom direction opposed to the top direction. The body comprises preferaby a top side and a bottom side 12. The horizonal plane is perpendicular to the vertical direction. The horizontal plane is spanned by a first direction and a second direction. The first direction extends from a first side 6 to a second side 7 of the body. The second direction extends perpendicular to the first direction and/or extends from a third side to a fourth side of the body. The terms horizontal and vertical shall not limit the invention on the final position of the body in the vagina. The body is preferably symmetrical to a symmetry plane parallel to a plane spanned by the vertical direction and the first direction. The symmetry plane in the sealing position in the vagina would correspond to the saggital plane of the woman wearing the intravaginal device.

The body comprises preferably a rim 4 and a closure means 2. The rim 4 is ring-shaped. The ring-shaped rim 4 extend preferably substantially in the horizontal plane. Preferably, the ring-shaped rim 4 extends fully in the horizontal plane, i.e. in a side view from the first, second, third or fourth side, the rim 4 would extend along a straight line. However, it would also be possible that the ring-shaped rim 4 is not fully extending in the horizontal plane, for example when the side view from the third or fourth side shows the rim 4 from the first side 6 to the second side 7 (at least in parts) as a curved or bent line, especillay with the second side 7 of the rim 4 bending or curving more towards the top direction out of the horizontal plane. This could have the advantage to arrange the second side higher 7 closer to the posterior cervix. In this case, the horizontal plane would be considered the horizontal plane of the principal extension of the rim 4.

The ring-shape of the rim 4 shall include any shape of the rim 4 in which the trajectory of the rim 4 meets again with a preivous point of the trajectory of the rim 4 to form a ring and/or to enclose a surface. Preferably, one end of the rim 4 is connected with the opposed end of the rim 4 so that the rim 4 is basically endless as the rim 4 does not end at one end, but start right over again. The two ends could be integrally formed or could be connected. In case, the two ends are integrally formed, i.e. the ring-shaped ring is manufactured already as a ring, the above-mentioned ends shall be considered as virtual ends which could be any point along the trajectory of the ring-shaped rim 4. Thus, this point would form the two virtual ends of the rim 4 meeting together. Preferably, the two ends of the rim 4 meet such that the cross-section of the rim 4 at the first end touches the cross-section of the rim 4 at the second end, preferably such that the cross-sections are aligned and/or that the cross-section of the first end does not protrude the cross-section of the second end. However, it would also be possible that the first end has a first end portion and the second end has a second end portion which touch each other to close the ring. For example, the first end portion and the second end portion could be arranged parallel to each other touching each other in this parallel arrangement.

The ring-shape of the rim 4 encloses a surface which has preferably a typical shape of vaginal wall in the sealing position of the device. The ring-shape of the rim 4 is preferably round with a longitudinal axis parallel to the first direction longer than a lateral axis parallel to the second direction (see first to sixth embodiment). Preferably, the ring-shape of the rim 4 is an oval ring-shape, i.e. the ring of the rim 4 encloses an ovally formed surface. The oval ring-shape rim 4 can have an egg ring-shape, an elliptic ring-shape, a Cassini oval ring-shape, a stadium ring-shape or many more oval shape. However, it is also possible that the ring-shape of the rim 4 is circular as in the seventh embodiment. The ring-shape of the rim 4 refers to the shape of the ring-shaped rim 4 from a top view or a view on the ring from a view direction which is perpendicular to horizontal plane. The centre point of the rim 4 is the centre point of the ring formed by the rim, e.g. the intersection point of the longitudinal and lateral axis. It is clear from the previous definition of the term "ring-shape" that this is not limited to circular rings and also other ring-shapes, e.g. oval ring shapes shall be covered by this term.

The rim 4 is configured to be in contact with the vaginal wall 28, when the body is in the sealing position. The rim 4 is configured to press against the vaginal wall 28 to obtain the sealing effect. The rim 4 is preferably thicker than the closure means 2 so that the rim 4 is more rigid than the closure means 2 and exerts a stronger force against the vaginal wall 28. The rim 4 could comprise one or more sealing lips extending as a closed ring at the outside of the rim 4 to improve the sealing effect (not shown in the figures).

To insert the body into the vagina, two opposed sides of the rim 4 are preferably pressed together so that they extend basically parallel. Preferably the third side and the fourth side of the rim 4 are pushed together, when inserting the body in the vagina through the opening 23. If the compressed body is then inserted with the second side towards the cervix 27, the second side 7 of the rim 4 will be automatically positioned at the posterior cervic 27, if the first side 6 is pushed behind the pubic bone. By releasing the pressure on the third and fourth side of the rim 4, the rim 4 presses against the vaginal walls 28 and stabilizes in the sealing position. The pubic bone prevents that the rim 4 moves towards the opening 23 so that the body remains stably in the sealed position.

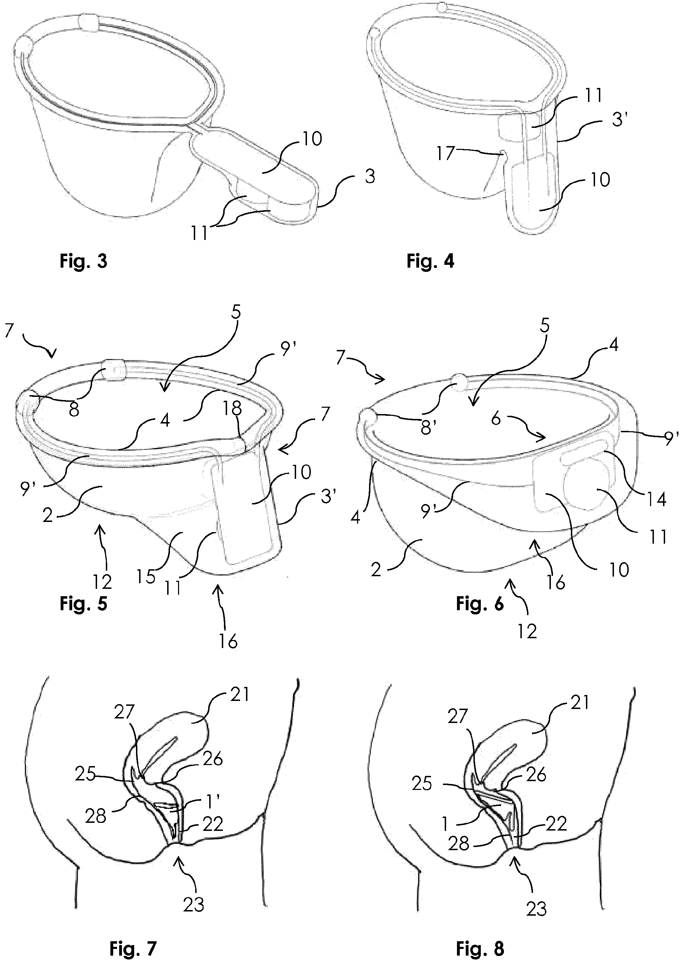

The rim 4 has preferably at least one bending point 18 at which the bending of the ring-shaped rim 4 is easier than at other parts of the rim 4. The bending point 18 is preferably arranged at the first side 6 and/or at the second side 7 of the rim 7. In the first and second embodiment of the intravaginal device 1, the bending point 18 is realized by a notch or a corner which facilitates the bending. In the third embodiment in Fig. 5, in addition to the the notch or corner, the rim 4 is cut so that the rim 4 opens when the third and fourth side are compresses one versus the other. Since the rim 4 in the sealing position pushes against the vaginal wall 28, this force against the wall 28 is also exterted at the bending point 18 where the two ends of the rim 4 push one versus the other and assure that the sealing is tight enough.

The closure means 2 is configured to close the surface area within the ring-shaped rim 4 to block fluids through the vagina. Thus, the closure means is connected with its peripheral sides with the rim 4 in order to close the opening formed by the rim 4. In the intravaginal collecting hygiene devices according to the shown embodiments, the closure means 2 is a recipient or container for collecting vaginal fluids. The recipient extends from the rim 4 or the horizontal plane downwards (in the bottom direction). The recipient has an opening 5 on its top side. In the sealing position, the vaginal fluids flow into the opening 5 of the recipient 2 and are collected in the recipient 2. Since the rim 4 seals the body versus the vaginal wall 28, the vaginal fluids can only flow into the recipient 2. The recipient contains preferably lateral walls which extend at least partly in the bottom direction until the bottom side 12 of the recipient 2 or of the body. Obviously, the closure means 2 could also first extend in the horizontal plane towards the centre of the ring-shaped rim 4 and then downwards in the recipient 2. The recipient axis is the line between the deepest point of the recipient and the centre point of the rim 4. If the recipient comprises a region (not a point) which is deepest, the centre point of the deepest region is considered as the deepest point for the definition of the recipient axis. The recipient axis could extend along the vertical axis. The recipient axis could also have an angle with the horizontal plane larger than 45°, preferably larger than 60°, preferably larger than 75°, preferably larger than 80°. The recipient axis could have an angle with the first direction (angle enclosed between first side of rim 4, center point of rim 4 and deeest point of bottom side 12) could be made smaller than 90° in order to adapt the recipient 2 to the anatomy of the cervix 27, when the body is positioned in the vagina. While these assymmetries between the first and second side 6, 7 of the body are advategoues for the first to sixth embodiment which like a menstrual disc are positioned between the pubic bone and the posterior cervix 25, the seventh embodiment which is positioned like a menstrual cup rather in the vaginal canal is rather symmetric and the recipient axis extends preferably in the vertical direction or is perpendicular to the rim 4 or its horizontal plane. The recipient 2 has preferably a capacity to collect a volume of more the 10 millilitre (ml), preferably more than 20 ml, preferably more than 30 ml, preferably more then 35 ml. The recipient 2 has preferably a capacity to collect a volume of less than 100 millilitre (ml), preferably less than 90 ml, preferably less than 80 ml, preferably less than 70 ml.

Fig. 7 and 8 show the anatomy of the vagina of the woman. The vagina comprises an opening 23 at one end and the cervix 27 as the other end of the vagina and as the entrance of the uterus 21. The vaginal canal 22 connects the opening 23 and the cervix 27. The side of the vagina arranged towards the rectum is the posterior side of the vagina and the side of the vagina arranged towards the bladder is the anterior side of the vagina. Vaginal walls 28 surround the vaginal canal 22 from the entrance 23 up to the cervix 27. The posterior vaginal walls 28 close to the cervix 27 are called the posterior cervix 25. The anterior vaginal walls 28 close to the cervix 27 are called the anterior cervix 26. The pubic bone is arranged in the anterior vaginal wall 28 of the vagina. The pelvic nerves pass close to the posterior cervix 25. This is why the electrostimulation for treating palvic nerves are most effective close to the posterior cervix 25.

As already mentioned, the first to sixth embodiments of the intravaginal device 1 are configured to be positioned at the upper end of the vaginal canal close to the cervix 27. Preferably, the intravaginal device 1 is positioned with its second side 7 at the posterior cervix 25 (similar to a menstrual disc). The first side 6 of the intravaginal device 1 is positioned preferably at or above the pubic bone. This allows to fix the intravaginal device 1 between the anterior vaginal wall, preferably the pubic bone and the posterior cervix 25 as shown in Fig. 8.

In an alternative embodiment of the intravaginal device 1', it is positioned rather in the lower vaginal canal 22 as shown in Fig. 7 (similar to a menstrual cup).

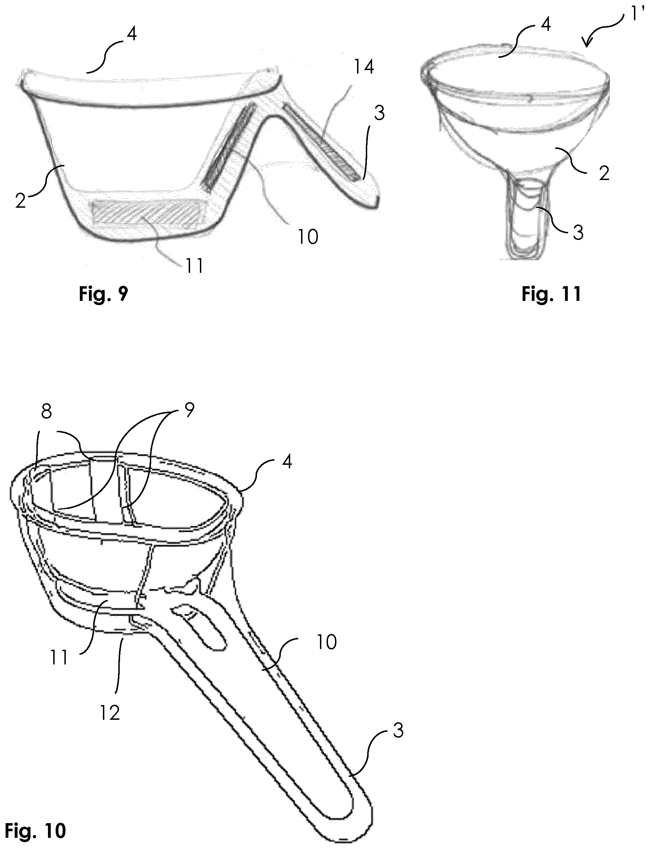

In a preferred embodiment, the intravaginal device 1, 1' comprises a removal protrusion 3. The removal protrusion is configured to facilitate the removal of the intravaginal device 1, 1' from the vagina. The removal protrusion 3 extends/protrudes preferably from the second side 7 and/or from the rim 4 of the body (see first to fourth embodiment and sixth embodiment. The removal protrusion 3 could extend (in its non-compressed state) in the horizontal plane and/or radially with respect to the centre point of the rim 4 as for example in the first embodiment in Fig. 1 or 3. The removal protrusion 3 could extend more downwards from the horizontal plane like the second, third, fourth and fifth embodiment. The removal protrusion 3 is preferably bendable at the connection point/line with the rim 4. This facilitates the insertion of the body. The removal protrusion 3 is in some embodiment not bendable versus the top direction. This can be achieved by side-flaps 15 which connect the removal protrusion 3 laterally to the removal protrusoin 3 as shown for example in Fig. 5. The side flaps are connected on the other side with the lateral wall of the recipient 2 and holding the removal protrusion 3 down. This creates a kind of pocket 16 (between the removal protrusion 3, the lateral wall of the recipient 2 and the flaps 15) for inserting a finger th grap the removal protrusion 3. In fig. 6, a similar pocket 16 is achieved by increasing the rim 4 on the second side 7 and form the removal protrusion 3 with this increased rim 4 (similar to collar of a pullover). This limits the movement of the removal protrusion in the top direction (or at least makes the movement towards the top direction (further away from the recipient 2) harder than versus the down direction (closer to the recipient 2)). In some embodiments, e.g. in Fig. 10 and 11, the removal protrusion extends from the bottom side 12 of the recipient 2. In some embodiment, e.g. in Fig. 11, the removal protrusion 3 extends along the recipient axis or vertical axis from the bottom side 12 of the recipient. In some embodiment, e.g. in Fig. 10, the removal protrusion 3 extends along the first direction from the bottom side 12 of the recipient (maybe with a component towards the vertical direction).

The electrostimulation means are configured to stimulate the vaginal wall 28 (preferably the posterior cervix 25) to treat pelvic pain of the woman. The electrostimulation means (electronics) comprise electrodes 8, signal generation means 10, conductors 9 to connect the electrodes 8 with the signal generation means 10 and a power source 11.

The electrodes 8, 8' are preferably two electrodes. However, in a different embodiment, it would also be possible to have one electrode or more than two electrodes. The electrodes 8, 8' are preferably arranged in the rim 4. The electrodes 8 are preferably arranged on the second side 7 of the body, preferably at the second side 7 of the rim 4. If the body in the sealing position, i.e. in the position in which the body should be placed in the vagina, is arranged with its second side 7 at the posterior cervix 25, the electrodes at the second side 7 of the rim 4 would generate a good contact with the vaginal wall 28 at the posterior cervix 25 due to the sealing pressure generated by the rim 4. In one embodiment, the electrodes 8 could be realized as conductive silicon embedded in (non-conductive) silicon of the rest of the rim 4 (see embodiments of Fig. 1, 3, 5, 9). The conductive silicon is connected with the conductor 9 to receive the electrostimulation signal from the electronics. In another embodiment, the electrodes 8' are realized as metal elements on the surface of the rim 4 connected with the conductors 9.

The signal generator means is configured to generate the electrostimulation signal which is applied to the electrodes 8 to treat the pelvic pain with electrostimulation. The signal generator means comprises preferably a chip configured to generate the electrostimulation signal. Preferably, the signal generator means comprises preferably an analouge circuitry to transfer the digital electrostimulation signal in an electric signal. This analogue circuitry could comprise an amplifier. However, it would also be possible to generate the signal fully analoguously and remove the need of a chip. The signal generator means is preferably realized in a circuit board preferably a printed circuit board (PCB) 10. The PCB could be realized as a flex PCB, a rigid PCT or a rigid-flex PCB. The flex PCB 10 has the advantage that the PCB is not rigid and increases the wearing comfort. The flex PCB 10 can be further used to realize the conductors 9 as conductor tracks from the signal generator to the electrodes 8. For example, in the realizations in Fig. 1, 3, 5 and 10. The conductor tracks 9 on the flex PCB acting as conductors 9 can be arranged in the rim 4 to connect the electrodes 8 at the second side 7 of the rim 4 with the signal generator at the first side 6, e.g. in the removal protrusion 3. However, the conductor tracks of the flex PCB could also be arranged in the lateral wall of the recipient 2 to connect the electrodes 8 at the second side 7 of the rim 4 with the signal generator or power source 11 in the bottom 12 of the recipient 2. However, the PCB 10 could however also be realized as a rigid PCB or as a rigid-flex PCB, the latter comprising rigid portions and flexible portions.

The signal generator means or the PCB 10 is preferably arranged in the removal protrusion 3 and/or at the second side of the device 1. However, it is also possible to arrange the signal generator means or the PCB 10 in the lateral wall of the recipient as in Fig. 9 or in the bottom 12 of the recipient 2 as in Fig. 10 or in the rim 4 (not shown).

The design of the stimulation signal to treat pelvic pain is known in the state of the art and is described subsequently only shortly without any limitation of the invention. Electrostimulation signals used in intravaginal devices for treating pelvic pain are carefully designed to deliver therapeutic effects while ensuring comfort and safety. These signals typically feature a biphasic waveform, which prevents tissue irritation by balancing the electrical charge, and are often delivered in a pulsed pattern to mimic natural nerve activity. The frequency range can be selected or adjusted based on the therapeutic goal. The amplitude, measured in milliamps, is customizable and generally kept low, ensuring gentle stimulation suitable for the sensitive posterior cervix. Pulse widths, typically between 50 to 400 microseconds, are chosen to either target sensory nerves or penetrate deeper tissues for muscle relaxation. To enhance user safety, these devices include features like automatic shut-off after a session, gradual ramp-up of intensity, and safeguards against electrical overload. Overall, these signals aim to modulate pain, relax pelvic muscles, and improve local circulation, providing targeted and effective relief for pelvic pain.

One embodiment, could allow to change the stimulation signal based on a user input. The user input could be received based on a haptic user input on the device 1 itself. In a preferred embodiment, the user input to select or to receive the stimulation signal is received wirelessly via a communication interface (see description later). The device 1 could offer multiple modes, such as continuous or burst delivery, to accommodate different user needs and treatment protocols. The selection could be done by a switch in the device 1 or received via the wireless communication interface.

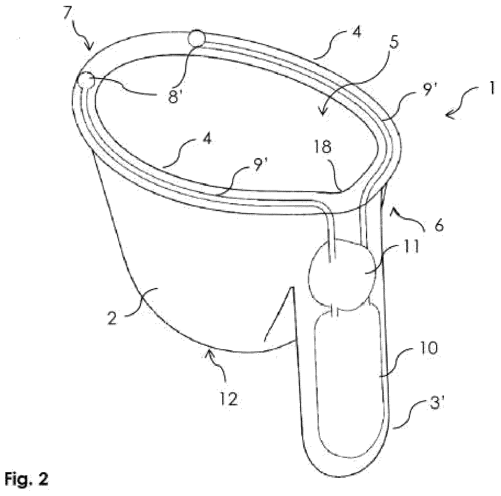

The power source is preferably a battery 11. The battery 11 is preferably arranged in the removal protrusion 3. However, it is also possible to arrange the battery 11 somewhere else in the body/device 1, e.g. in the bottom 12 as in fig. 9 and 10. The battery 11 or other power source can be mounted on the PCB 10 (see Fig. 5, 6) or could be connected by wires (see e.g. Fig. 2, 4) or by flex PCB conductor tracks (see e.g. Fig. 1, 10). The electronics comprise preferably a charging circuit for the wirelessly charging the battery 11. This allows to encapsulate the electronics for good cleaning of the device 1 and at the same time guaranteeing a proper charging of the battery 11.

The electronics comprise preferably a wireless communication interface, e.g. a bluetooth interface. The wireless communication interface could be integrated on the PCB 10, e.g. as a communication chip mounted on the PCB 10, and an antenna 14. The antenna 14 can be realized on the PCB 10 as well or as an extra component 14 like in Fig. 1, 6, 9. The wireless communication interface could be used to control the electrostimulation means, e.g. via a connected smartphone or other computing device. The electrostimulation means could be controled via a user input from an app on a smartphone. The user input can be used to switch the electrostimulation on and off. It could be further used to change the type of electrostimulation signal. The wireless communication interface could further give out certain information about the device 1, for example the state of the battery 11. It is also possible to include a filling state sensor of the receiptable to give out over the wireless communication interface when the collecting hygenic device 1 is full and must be emptied.

In the first embodiment shown in Fig. 1, the signal generator means or the PCB 10 and the battery 11 are arranged in the removal protrusion 3. The removal protrusion 3 extends in the (horizontal) plane of the ring-shaped rim 4 (radially from the rim 4) at the first side 6 opposed to the electrodes 8. The connection point between the rim 4 and the removal protrusion 3 is very thin and/or provides a maximum flexibility/bendability of the removal protrusion with respect to the rim 4. A hard PCB 10 is connected via a conductor 9 with th electrodes. A first conductor 9 extendes from the PCB 10 of the removal protrusion 3 through one side (on third side of device 1) of the rim 4 to the first electrode 8. A second conductor 9 extendes from the PCB 10 of the removal protrusion 3 through the other side (on fourth side of device 1) of the rim 4 to the second electrode 8. The conductor 9 is realized here as two conductor tracks 9 extending on a flex PCB. The flex PCB has a first portion which is arranged in the removal protrusion 3 with the first and second conductor tracks. The first portion of the flex PCB bifurcates into two arms, with each arm extending on opposed sides of the rim 4 to the respective electrodes. When the width of each arm is small, the arm behaves in flexibility terms like a wire. Thus, the first conductor track extends on the first arm to the first electrode 8 and the second conductor track extends on the second arm to the second electrode 8. However, the first and second arm could be embedded in the (silicon) rim 4 so that the rim material insulates the conductor tracks 9. The flex PCB with the conductor tracks 9 could be connected to a harc PCB 10 or the rigid PCB 10 and the flex PCB with the conductor tracks 9 could be realized as a rigid-flex PCB. It is also possible to realize the PCB 10 as flex PCB as well and to make the signal generator and the conductor tracks 9 all on the flex PCB. The first portion of the flex PCB 9 connects preferably also the battery 11. However, the conductors 9 could also be realized as wires 9' as in Fig. 3. The electrodes 8 are preferably realized as conductive silicon. The conductor track 9 ends in the portion of the rim 4 with the electrodes 8. In these portions, the conductor track 9 or its flex PCB is embedded in a conductive silicon so that the stimulation signal from the conductor track 9 is applied on the respective electrode 8. However, it is also possible to use metal electrodes as in Fig. 3. The metal electrodes could also be combined with the conductor tracks of the flex PCB or the wires 9' of Fig. 3 with the electrodes 8 realized as conductive silicon.

Fig. 3 shows a variation of the first embodiment in which the battery 11 is mounted directly on the PCB 10.

Fig. 2 shows a second embodiment of the intravaginal device 1. The removal protrusion 3' in the second embodiment downwards or substantially parallel to the recipient axis. The recipient 2 is also connected to the removal protrusion 3 so that the removal protrusion 3' is prevented to be bent upwards. Preferably, the side of the removal protrusion 3' pointing away from the recipient 2 is connected to the rim 4. Preferably, the side of the removal protrusion 3 pointing towards the recipient 2 is connected to the lateral wall of the recipient 2. Here, the electrodes 8' are realized as metal electrodes 8', even though if also other electrodes could be used, e.g. the conductive silicon electrodes 8 from Fig. 1. The electrodes 8 or 8' are connected with wires 9' with the signal generator means or the PCB 10 in the removal protrusion 3'. The same wires 9' are used to connect the battery 11 in the removal protrusion 3' with the PCB 10. The first wire extends from the first electrode 8' through the (third side of the) rim 4 to the removal protrusion 3'. The second wire extends from the second electrode 8' through the (third side of the) rim 4 to the removal protrusion 3'.

In the variant of the second embodiment shown in Fig. 4, the cylindrical battery is arranged in a rotated position. In Fig. 3, the cylinder axis is arranged in the first direction, while in Fig. 4, the cylinder axis of the battery 11 is arranged parallel to the vertical direction or to the direction of the removal protrusion 3' or to the direction of the recipient axis. Another small difference is that along the connection line of the lateral wall of the recipient 2 and the side of the removal protrusion 3' pointing towards recipient 2, the thickness of the removal protrusion 3' is reduced by a recess or notch 17. This increases the capability of the removal protrusion 3' to bend and might facilitate the removal of the body from the vagina.

While the recipients 2 in the first and second embodiment were designed for receiving 60 ml, the recipients in the third and fourth embodiment are designed smaller, here for 40 ml.

The third embodiment in Fig. 5 has a removal protrusion 3' similar as in the second embodiment. The removal protrusion 3' is additionally connected at its lateral sides with the lateral wal of the recipient 2. This forms a pocket 16 between the lateral wall of the recipient 2, the flaps 15 and the removal protrusion 3'. Here the battery 11 is mounted directly on the PCB 10 arranged in the removal protrusion 3'. Since the battery 11 points towards the pocket 16, the component does not create discomfort for the woman. In the third embodiment, the electrodes are realized as conductive silicon as in the first embodiment in Fig. 1 and the conductor 9' as wires as in the second embodiment in Fig. 2.

In the fourth embodiment in Fig. 6, the rim 4 starts from the third and fourth side to extend more downwards the closer you get to the first side 6 of the rim. The rim 4 extending downwards is a bit like a collar which extends parallel to the lateral wall of the recipient 2. Thus, the rim 4 on the first side 6 forms thus a removal protrusion 3 and/or generates a pocket 16 between the lateral wall of the recipient 2 and the rim 4 or the removal protrusion 3. The electrodes 8' are realized as metal electrodes 8' and the conductors 9' as wires. The PCB 10 with the battery 11 and the antenna 14 are arranged in the lateral wall of the recipient at the first side 6 so that the components of the PCB are pointing in the pocket 16.

In the fifth embodiment in Fig. 9, the battery 11 is arranged in the bottom 12 of the recipient 2, the PCB 10 is arranged in the lateral wall of the recipient 2 on the first side 6. Preferably, the antenna 14 is arranged in the removal protrusion 3.

In the sixth embodiment in Fig. 10, removal protrusion 3 extends not from the rim 4, but from the bottom side 12 of the reicipient 2. This allows to arrange parts of the electronics in the bottom side 12 of the recipient 2 and the removal protrusion 3 and to connect the electrodes 8 at the second side 6 with conductors 9 extending from the bottom side 12 to the second side 7 of the rim 4 along the lateral wall of the recipient 2. For example, in the shown embodiment, the battery 11 is arranged in the bottom 12 of the recipient 2 as in Fig. 9 and/or the PCB 10 is arranged in the removal protrusion 3. The PCB 10 and the conductors 9 are arranged by one flex PCB.

The seventh embodiment in Fig. 11 shows an intravaginal device 1' which is positioned more towards the opening 23 of the vagina compared to the intravaginal devices 1 shown in Fig. 1 to 6 and Fig. 8 to 10. The intravaginal device 1' is positioned in the vaginal canal 22 (below the cervix) as shown in Fig. 7. The shape of the intravaginal device 1 ' is like a menstrual cup and/or is bell or funnel shaped. The removal protrusion 3 is arranged at the bottom side 12 at the centre of the recipient 2 extending in the vertical direction. This embodiment might have some advantages in terms of comfort as it is not inserted that deep, but has some disadvantageous for the pelvic pain treatment as the electrodes are farther away from the posterior cervix 25.

The intravaginal device was described as a intravaginal collecting hygiene device. However, it is also possible that the intravaginal device is a contraceptive device as a contraceptif diaphragma or a contraceptif cup with the electrostimulation means as described above. Similar as the intravaginal device device described, the contraceptif device would have a rim and electrodes arranged in the rim of the contraceptif cup or diaphragma.

It should be understood that the present invention is not limited to the described embodiments and that variations can be applied without going outside of the scope of the claims.

## Claims

1. Intravaginal device for treating pelvic pain and for blocking fluids through a vagina, the intravaginal device compr<ises: a body configured to be placed in a vagina, the body comprising a ring-shaped rim configured to seal the body against the vaginal wall, when placed in the vagina, the body comprising a closure mean closing the surface area within the ring-shaped rim to block fluids through the vagina; and electrostimulation means configured to stimulate the vaginal wall to treat pelvic pain.

2. Intravaginal device according to claim 1, wherein the body comprises a rim configured to be placed/pushed against a vaginal wall of the vagina and a blocking means in the space enclosed by the rim (to block the passage through the vagina at the position of the rim in the vaginal channel).

3. Intravaginal device according to claim 1 or 2, wherein the intravaginal device being a collecting menstrual hygiene device and/or wherein the closure means forms a receiptable to collect vaginal fluids.

4. Intravaginal device according to claim 3, wherein the intravaginal device being a menstrual disc.

5. Intravaginal device according to claim 3 or 4, wherein the closure means forms a receiptable to collect vaginal fluids.

6. Intravaginal device according to one of claims 3 to 5, wherein the closure means or body has a diameter smaller than 80 mm and larger than 50mm.

7. Intravaginal device according to one of claims 2 to 6, wherein the body is designed such that a first side of the rim can be positioned at a fornix of the vagina sealing the vagina.

8. Intravaginal device according to one of claims 2 to 7, wherein the body comprises a removal protrusion for facilitating the removal of the intravaginal device.

9. Intravaginal device according to claim 8, wherein the removal protrusion protrudes from the rim.

10. Intravaginal device according to claim 9, wherein the removal protrusion is attached bendable at the rim so that the angle between removal protrusion and an axis of the receptable is changeable.

11. Intravaginal device according to one of claims 1 to 10, wherein the electrostimulation means comprises two electrodes arranged in the rim.

12. Intravaginal device according to claim 11, wherein the body is made off silicon, wherein the at least one electrode is realized in conductive silicon embedded in the silicon of the body.

13. Intravaginal device according to claim 10 or 11, wherein the electrostimulation means comprises a power source, electronics and a conductor means, wherein the conductor means are configured to connect the electronics with the at least one electrode, wherein the electronics are configured to receive the power from the power source and to generate an electrical stimulation signal at the at least one electrode to stimulate the vaginal wall.

14. Intravaginal device according to claim 13, wherein the conductor means extends along the rim.

15. Intravaginal device according to one of claims 13 to 14, wherein the electronics and/or the power source is arranged in the removal protrusion.
